# EUROPEAN PATENT APPLICATION

(11) **EP 4 674 475 A1**
(43) Date of publication of application: **07.01.2026**
(21) Application number: 24764250.7
(22) Date of filing: 26.02.2024
(51) Int. Cl.: A61N 1/40, A61N 1/06, A61N 1/32, A61N 1/36

(54) **HIGH-FREQUENCY SKIN TREATMENT DEVICE AND METHOD OF CONTROLLING SAME**

(30) Priority: 27.02.2023 KR 20230025676
(71) Applicant: CLASSYS INC., Seoul 06220 (KR)
(72) Inventor: CHEON, Yeong Sook, Suwon-si Gyeonggi-do 16583 (KR); NAM, Su Hee, Seoul 02637 (KR)
(74) Representative: Witte, Weller & Partner Patentanwälte mbB
(86) International application number: PCT/KR2024/095446
(87) International publication number: WO 2024/181852

(57) **Abstract**

The purpose of the present invention is to provide a high-frequency skin treatment device and a method of controlling same, wherein the device performs high-frequency skin treatment by transferring high-frequency energy to skin, and high-frequency invasive treatment and non-invasive treatment can be performed selectively or simultaneously by the single device. The device can improve the efficiency of high-frequency energy transfer and reduce the patient's fear and pain due to the invasion, by performing the invasive treatment and non-invasive treatment simultaneously, thereby enabling comfortable treatment.

## Description

### [Technical Field]

The present disclosure relates to a high-frequency skin treatment device and a method of controlling the high-frequency skin treatment device. More particularly, the present disclosure relates to a skin treatment device that enables achieving effects such as improving wrinkles of the skin and enhancing skin elasticity using high-frequency energy by selectively applying an invasive method or a non-invasive method or by applying the two methods in combination to the skin, and a method of controlling the high-frequency skin treatment device.

### [Background Art]

The human skin is composed of the epidermis, the dermis, and the subcutaneous fat layer beneath them, and the dermis beneath the epidermis is connected to a basal layer and contains water, proteins, carbohydrates, and minerals in a gel-like state.

The dermis of the skin is composed of a papillary layer, which contains capillaries that provide nutrients to the epidermis and are associated with blood circulation and lymphatic vessels that transport lymph, and a reticular layer composed of collagen that forms collagen fibers associated with skin wrinkles, elastin that forms elastin fibers providing skin elasticity, and an extracellular matrix.

For skin aesthetics such as the prevention of skin aging, beneficial substances are delivered to the dermis described above or synthesis of new collagen is induced. As a representative procedure method for such skin aesthetics, a method that inserts microneedles into the dermis of the skin, forms a coagulation region in a specific area of the dermal tissue by applying radiofrequency energy to the dermal tissue, and then induces synthesis of new collagen in the dermis during a natural healing process of the coagulation region is used.

For example, a plurality of prior art references such as U.S. Patent Application Publication No. US2005-0222565, U.S. Patent No. US9,095,357, U.S. Patent No. US9,510,899, U.S. Patent No. US6,277,116, and U.S. Patent Application Publication No. US2002-0128641 discloses a device and a method that form a thermal coagulation region in the dermal tissue by inserting a plurality of microneedles into the dermis of the skin and then applying RF energy to the microneedles.

On the other hand, the invasive method using microneedles as described above has an advantage that the needles can directly penetrate the dermis and deliver radiofrequency energy, but since it is accompanied by the patient's fear and pain due to the invasiveness, a device that delivers radiofrequency energy in a non-invasive manner to obtain an effect similar to that of the invasive method is used.

For example, Korean Patent Application Publication No. 2005-0114676 and U.S. Patent Application Publication No. US2014-0128944 discloses a non-invasive device that brings an electrode at the tip into contact with the skin and delivers radiofrequency energy to the skin through the electrode.

However, since the non-invasive radiofrequency device as described above generates heat with the electrode in contact with the epidermis of the skin using radiofrequency energy, it has a high risk of causing epidermal damage due to burns, while it is difficult for the thermal energy generated by the radiofrequency to be effectively delivered to the dermis beneath the epidermis, so that there is a disadvantage that a long-term procedure is required to obtain the desired effect of skin treatment.

That is, in a skin treatment method using radiofrequency energy, the invasive method and the non-invasive method as described above each have advantages and disadvantages, and which method is used for performing the procedure may be selectively determined.

However, invasive radiofrequency treatment devices and non-invasive radiofrequency treatment devices are implemented as completely separate independent devices or as separate independent handpiece devices in a single main body, and thus there has been an inconvenience that it is required to individually operate corresponding devices when performing invasive treatment and when performing non-invasive treatment.

Further, in performing radiofrequency skin treatment as described above, since it is required to individually operate different respective devices, there is a problem in the related art that rapid switching between invasive treatment and non-invasive treatment is difficult.

In addition, even if treatment is to be performed by simultaneously carrying out invasive treatment and non-invasive treatment, the there is a problem that it cannot be realized in the related art described above.

### [Disclosure]

### [Technical Problem]

An objective of the present disclosure is to provide a high-frequency skin treatment device that performs radiofrequency skin treatment by delivering radiofrequency energy to the skin, and a method of controlling the high-frequency skin treatment device, the high-frequency skin treatment device being capable of selectively or simultaneously performing invasive treatment and non-invasive treatment through one device, and improving the efficiency of radiofrequency energy delivery and reducing the fear and pain of a patient due to invasiveness to enable comfortable treatment by simultaneously performing invasive treatment and non-invasive treatment.

### [Technical Solution]

A high-frequency skin treatment device according to an embodiment of the present disclosure includes: a contact electrode unit configured to be brought into contact with a treatment surface of a skin and generate deep heat in a deep region of the treatment surface by applying a radiofrequency signal; a needle assembly including a plurality of needle electrodes configured to penetrate the treatment surface and deliver heat to tissue using a radiofrequency signal; a needle driving unit configured to control insertion of the plurality of needle electrodes by moving the needle assembly; a multi-channel radiofrequency generator including a first channel configured to transmit a radiofrequency signal for generating the deep heat to the contact electrode unit, and a second channel configured to transmit a radiofrequency signal for heating the tissue to the plurality of needle electrodes; and a controller configured to preset a plurality of treatment modes according to any one of or a combination of non-invasive treatment using the contact electrode unit and invasive treatment using the plurality of needle electrodes, and to control the needle driving unit and the multi-channel radiofrequency generator in accordance with selected treatment modes.

Further, preferably, the controller, in accordance with the treatment modes, may be configured to control a generation depth of deep heat in the skin by the contact electrode unit by switching a radiofrequency signal by controlling the first channel of the multi-channel radiofrequency generator in the non-invasive treatment, and to control a tissue heating depth of the inserted needle electrodes by controlling an insertion depth of the plurality of needle electrodes into the skin by controlling the needle driving unit in the invasive treatment.

Further, preferably, the controller, in accordance with the treatment modes, may control the first channel of the multi-channel radiofrequency generator to transmit a radiofrequency signal of a frequency for generating deep heat by the contact electrode unit at a depth corresponding to an insertion depth of the needle electrodes selected or preset for the invasive treatment in the non-invasive treatment, and, after deep heat is generated by the contact electrode unit, may control the needle driving unit to insert the plurality of needle electrodes to the selected or preset insertion depth of the needle electrodes such that the invasive treatment is performed.

Further, preferably, the first channel of the multi-channel radiofrequency generator may include: a first radiofrequency generator configured to generate a plurality of radiofrequency signals of different frequencies, depending on a generation depth of deep heat in a deep region inside the skin; and a switching module configured to switch between radiofrequency signals such that a radiofrequency signal of a frequency selected from the plurality of radiofrequency signals generated by the first radiofrequency generator is transmitted to the contact electrode unit.

Further, preferably, the needle driving unit may be configured to control a movement distance of the needle assembly under control of the controller such that an insertion depth of the plurality of needle electrodes into the skin can be adjusted; the first channel of the multi-channel radiofrequency generator may be configured to generate radiofrequency signals of a plurality of different frequencies depending on a generation depth of deep heat in the skin and to switch between the plurality of radiofrequency signals such that a radiofrequency signal of a selected frequency is transmitted to the contact electrode unit; the second channel of the multi-channel radiofrequency generator may be configured to generate a radiofrequency signal of a selected or preset frequency to transmit the radiofrequency signal to the plurality of needle electrodes such that the inserted plurality of needle electrodes delivers heat to tissue; and the controller may be configured to, when a frequency of the radiofrequency signal transmitted through the first channel is selected, perform control such that deep heat is generated in a depth region inside the skin corresponding to the selected frequency by the contact electrode unit, and to control the needle driving unit such that the plurality of needle electrodes is inserted to a needle insertion depth in the depth region, in which the deep heat has been generated, and the inserted needle electrodes transmit heat to the region in which the deep heat has been generated in accordance with the radiofrequency signal.

Further, preferably, the controller may be configured to, when a plurality of different frequencies for the non-invasive treatment is selected, separately control the first channel of the multi-channel radiofrequency generator, the needle driving unit, and the second channel of the multi-channel radiofrequency generator to sequentially perform a combined treatment of generating deep heat through the contact electrode unit, inserting the plurality of needle electrodes, and heating tissue through the inserted needle electrodes, at each of different depths in the skin corresponding in number to the number of the selected frequencies.

Further, preferably, the needle driving unit may be provided in a handpiece, and the needle assembly may be provided in a needle tip detachably connected to an end of the handpiece to be connected to the needle driving unit; the plurality of needle holes may be formed in a bottom surface of the needle tip such that the plurality of needle electrodes protrudes through the needle holes, respectively; and the contact electrode unit may include a first contact electrode and a second contact electrode that form an electrode pair around the plurality of needle holes on the bottom surface of the needle tip and protrude from the bottom surface of the needle tip.

Further, preferably, the needle driving unit may be provided in a handpiece, and the needle assembly may be provided in a needle tip detachably connected to an end of the handpiece to be connected to the needle driving unit; the plurality of needle holes may be formed in a bottom surface of the needle tip such that the plurality of needle electrodes protrudes through the needle holes, respectively; and the contact electrode unit may include a plurality of individual contact electrodes provided for the plurality of needle holes, respectively, to respectively come into contact with the peripheries of positions of the needle electrodes inserted in the treatment surface.

Meanwhile, a method of controlling a high-frequency skin treatment device according to an embodiment of the present disclosure is a high-frequency skin treatment device that provides treatment modes based on a combination of a non-invasive treatment for a skin that uses a contact electrode unit configured to come into contact with a treatment surface of the skin, and an invasive treatment that inserts a plurality of needle electrodes into the treatment surface and delivers heat to tissue in accordance with a radiofrequency signal. The method includes: selecting a frequency of a radiofrequency signal for the non-invasive treatment; generating deep heat in a depth region inside the skin corresponding to the selected frequency by applying the radiofrequency signal of the selected frequency to the contact electrode unit that is in contact with the treatment surface; controlling a needle driving unit to move the plurality of needle electrodes such that the plurality of needle electrodes are inserted to a needle depth in the depth region in which the deep heat has been generated; and applying a preset or selected radiofrequency signal to the plurality of needle electrodes such that heat is delivered through the plurality of needle electrodes to tissue at the needle depth in the depth region in which the deep heat has been generated.

Further, preferably, the method may further include: switching the frequency applied to the contact electrode unit to a radiofrequency signal of another frequency to change the depth region in which the deep heat has been generated; and controlling the needle driving unit such that the plurality of needle electrodes is inserted to a needle depth in the depth region corresponding to the frequency changed by the switching.

Meanwhile, a method of controlling a high-frequency skin treatment device according to another embodiment of the present disclosure is a high-frequency skin treatment device that provides treatment modes based on a combination of a non-invasive treatment for a skin that uses a contact electrode unit configured to come into contact with a treatment surface of the skin, and an invasive treatment that inserts a plurality of needle electrodes into the treatment surface and delivers heat to tissue in accordance with a radiofrequency signal. The method includes: setting the number of multi-shot stacks and selecting contact electrode unit application frequencies corresponding in number to the set number of the stacks; and delivering a radiofrequency signal to the contact electrode unit while switching a plurality of frequencies selected to generate deep heat in each of depth regions inside a skin corresponding to the set number of the stacks, setting a needle depth at which the plurality of needle electrodes is to be positioned, in each of the depth regions, and delivering heat according to a radiofrequency signal with the plurality of needle electrodes positioned at each of the needle depths under control of a needle driving unit.

Further, the delivering of heat according to a radiofrequency signal with the plurality of needle electrodes positioned at each of the needle depths may sequentially perform a combined treatment of generating deep heat through the contact electrode unit, moving the plurality of needle electrodes to a corresponding needle position, and heating tissue through the inserted needle electrodes, in each of the depth regions.

Further, preferably, in the selecting of contact electrode unit application frequencies, two may be selected as the number of the stacks, and a first frequency and a second frequency may be selected as the contact electrode unit application frequencies, and the delivering of heat according to a radiofrequency signal with the plurality of needle electrodes positioned at each of the needle depths may include: generating deep heat in a first depth region inside the skin by applying a radiofrequency signal of the first frequency to the contact electrode unit, inserting the plurality of needle electrodes to a first needle depth in the first depth region by controlling the needle driving unit, and delivering heat to tissue at the first needle depth by applying a radiofrequency signal to the inserted plurality of needle electrodes; and positioning the plurality of needle electrodes at a second needle depth in a second depth region corresponding to the second frequency by controlling the needle driving unit, generating deep heat in the second depth region inside the skin by switching the frequency of a radiofrequency signal applied to the contact electrode unit to the second frequency, and delivering heat to tissue at the second needle depth by applying a radiofrequency signal to the plurality of needle electrodes positioned at the second needle depth.

Further, preferably, in the selecting of contact electrode unit application frequencies, three may be selected as the number of the stacks, and a first frequency, a second frequency, and a third frequency may be selected as the contact electrode unit application frequencies, and the delivering of heat according to a radiofrequency signal with the plurality of needle electrodes positioned at each of the needle depths may include: generating deep heat in a first depth region inside the skin by applying a radiofrequency signal of the first frequency to the contact electrode unit, inserting the plurality of needle electrodes to a first needle depth in the first depth region by controlling the needle driving unit, and delivering heat to tissue at the first needle depth by applying a radiofrequency signal to the inserted plurality of needle electrodes; positioning the plurality of needle electrodes at a second needle depth in a second depth region corresponding to the second frequency by controlling the needle driving unit, generating deep heat in the second depth region inside the skin by switching the frequency of a radiofrequency signal applied to the contact electrode unit to the second frequency, and delivering heat to tissue at the second needle depth by applying a radiofrequency signal to the plurality of needle electrodes positioned at the second needle depth; and positioning the plurality of needle electrodes at a third needle depth in a third depth region corresponding to the third frequency by controlling the needle driving unit, generating deep heat in the third depth region inside the skin by switching the frequency of a radiofrequency signal applied to the contact electrode unit to the third frequency, and delivering heat to tissue at the third needle depth by applying a radiofrequency signal to the plurality of needle electrodes positioned at the third needle depth.

### [Advantageous Effects]

The high-frequency skin treatment device and a method of controlling the same according to the present disclosure, in a device that performs radiofrequency skin treatment by delivering radiofrequency energy to the skin, have the effect of selectively or simultaneously performing invasive treatment and non-invasive treatment through one device, and improving the efficiency of radiofrequency energy delivery and reducing the fear and pain of a patient due to invasiveness to enable comfortable treatment by simultaneously performing invasive treatment and non-invasive treatment.

### [Description of Drawings]

FIG. 1 is a diagram showing the configuration of a high-frequency skin treatment device according to an embodiment of the present disclosure.
FIG. 2 is a diagram showing a state in which needle electrodes are moved and protruded outward in the high-frequency skin treatment device shown in FIG. 1.
FIG. 3 is a block diagram showing the configuration and control system of the high-frequency skin treatment device according to an embodiment of the present disclosure.
FIG. 4 is a diagram showing the configuration of various types of contact electrode units in the high-frequency skin treatment device according to an embodiment of the present disclosure.
FIG. 5 is a diagram showing a configuration in which individual contact electrodes are provided as the contact electrode unit in the high-frequency skin treatment device according to an embodiment of the present disclosure.
FIGS. 6 and 7 are flowcharts showing a method of controlling the high-frequency skin treatment device according to an embodiment of the present disclosure, which are connected to each other on the basis of "A" to represent an entire single flowchart.
FIG. 8 is a diagram showing a case in which deep heat is generated at a deep position in the skin and thermal coagulation is generated by needle electrodes using the high-frequency skin treatment device according to an embodiment of the present disclosure.
FIG. 9 is a diagram showing a case in which deep heat is generated at a shallower position in the skin than the case shown in FIG. 8 and thermal coagulation is generated by needle electrodes.
FIGS. 10 and 11 are diagrams showing an example of a process of performing a multi-depth combined mode using the high-frequency skin treatment device according to an embodiment of the present disclosure.

### [Best Mode]

A high-frequency skin treatment device according to an embodiment of the present disclosure and a method of controlling the high-frequency skin treatment device are described in detail with reference to the drawings.

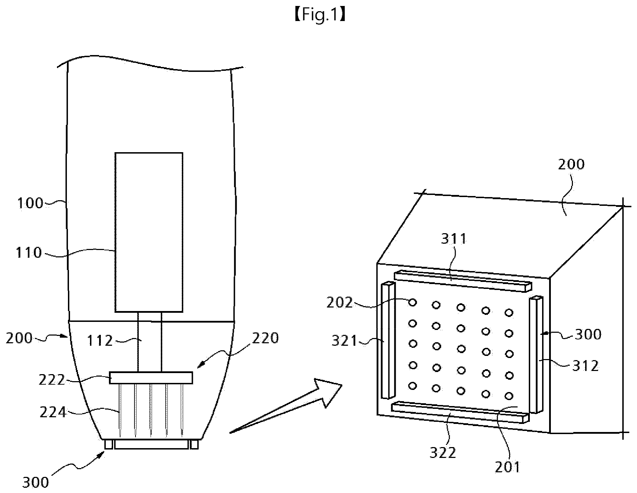

With reference to FIGS. 1 to 3, the configuration, operation, and control system of the high-frequency skin treatment device according to an embodiment of the present disclosure is described.

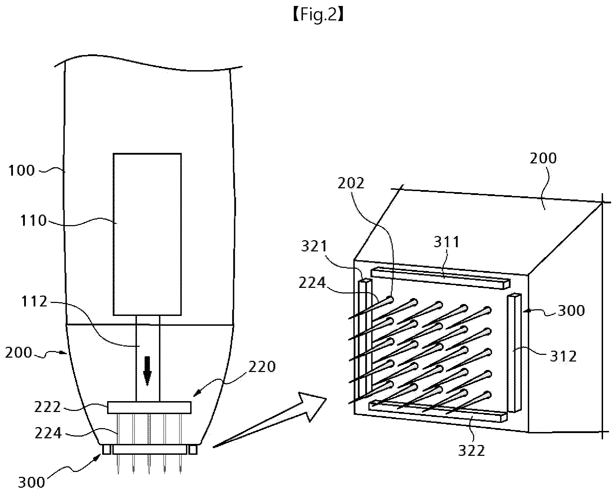

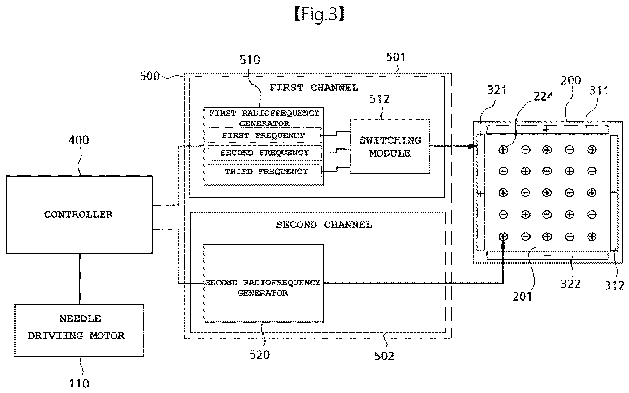

FIGS. 1 and 2 are diagrams showing the configuration and operation of the high-frequency skin treatment device according to an embodiment of the present disclosure, in which FIG. 1 shows a state before needles for invasive treatment are operated, and FIG. 2 shows a state in which the needles are operate and protruded outward. FIG. 3 is a block diagram showing the configuration and control system of the high-frequency skin treatment device according to an embodiment of the present disclosure.

As shown in FIGS. 1 and 2, a high-frequency skin treatment device according to an embodiment of the present disclosure include an applicator 100, 200 connected to a main device (not shown), and the applicator may include a handpiece 100 configured to be grasped by a practitioner and a needle tip 200 detachably connected to an end of the handpiece.

As shown in FIGS. 1 and 2, a needle assembly 220 may be provided inside the needle tip 200, a contact electrode unit 300 may be provided at an outer end of the needle tip 200, and a needle driving unit (including a driving motor 110 and a driving shaft 112) for moving the needle assembly 220 may be provided inside the handpiece 100.

The needle assembly 220 can be moved linearly in the vertical direction in the needle tip 200 by the needle driving unit, and may include a needle base 222 connected to the driving shaft 112 and a plurality of needle electrodes 224 fixed to the needle base 222. The needle electrodes 224 may be implemented as micro-needles having sharp ends and fine diameters to facilitate penetration into the skin.

Further, as shown in FIG. 1, a plurality of needle holes 202 formed at positions corresponding to respective needle electrodes 224 such that the plurality of needle electrodes 224 can protrude outward through the needle holes may be provided at an end of the needle tip 200.

When a practitioner operates an external switch (for example, a foot switch), the driving motor 110 in the handpiece 100 linearly drives the driving shaft 112 to move the needle base 222, and as the needle base 222 is moved, the plurality of needle electrodes 224 is moved, so that, as shown in FIG. 1, the plurality of needle electrodes 224 positioned in the needle tip 200 can protrude outward through the plurality of needle holes 202 of the needle tip 200 and, as shown in FIG. 2.

In a state in which the end of the needle tip 200 is in contact with the skin, through the process as described above, the plurality of needle electrodes 224 can protrude outward from the needle tip 200 and penetrate a treatment surface of the skin, and heat by a radiofrequency signal can be delivered to the dermal tissue inside the skin through the needle electrodes 224 that have penetrated the treatment surface of the skin. The plurality of needle electrodes 224 is electrically connected to a radiofrequency generator provided in the handpiece or in the main device, and each needle electrode can deliver heat to tissue through a radiofrequency signal generated by the radiofrequency generator. The generation and delivery of a radiofrequency signal by the radiofrequency generator will be described later.

Meanwhile, as shown in FIGS. 1 and 2, a contact electrode unit 300 provided at the end of the needle tip 200 is brought into contact with a treatment surface of the skin and generates deep heat in a deep region, that is, in tissue inside the skin of the treatment surface, through a radiofrequency signal.

As described above, skin invasive treatment by radiofrequency energy can be performed using the plurality of needle electrodes 224 of the needle assembly 220, and radiofrequency non-invasive treatment can be performed using the contact electrode unit 300 provided at the end of the needle tip 200.

That is, the high-frequency skin treatment device according to an embodiment of the present disclosure can provide a treatment mode for performing non-invasive treatment on the skin using a contact electrode unit that is brought into contact with a treatment surface of the skin, a treatment mode for performing invasive treatment by inserting a plurality of needle electrodes into a treatment surface of the skin and delivering heat to tissue in response to a radiofrequency signal, and a treatment mode according to a combination of the non-invasive treatment and the invasive treatment.

The contact electrode unit 300, which can perform non-invasive treatment on a treatment surface of the skin, may include a first contact electrode 311 and a second contact electrode 312 that form an electrode pair around the plurality of needle holes 202 on the bottom surface 201 of the needle tip 200 and protrude from the bottom surface of the needle tip 200.

The pair of the first contact electrode and the second contact electrode may be provided as only one pair at the end of the needle tip or may be provided as a plurality of pairs, and FIGS. 1 to 3 show a case in which two pairs of contact electrodes are provided.

In FIGS. 1 to 3, the first contact electrode 311 and the second contact electrode 312 are provided to be adjacent to each other, whereby they can form an electrode pair, and a third contact electrode 321 and a fourth contact electrode 322 are provided to be adjacent to each other, whereby they can form an electrode pair. Specifically, each contact electrode has a bar shape and may be disposed on each of the four sides of the bottom surface 201 of the needle tip 200.

In this context, the formation of an electrode pair by two contact electrodes means that the two contact electrodes have different polarities, and that when an alternating current is applied to the two contact electrodes, an electric field is formed between the two contact electrodes forming the electrode pair.

As shown in FIGS. 1 to 3, the first contact electrode 311 and the second contact electrode 312 may be arranged in the shape of "¬" while forming an electrode pair, and the third contact electrode 321 and the fourth contact electrode 322 may be arranged in the shape of "∟" while forming an electrode pair.

When a radiofrequency signal is applied, the electrode pair forms an electric field and generates deep heat in the deep region of a treatment surface of the skin with the contact electrode unit, which forms one or more electrode pairs as described above, in contact with the treatment surface of the skin, and the lower the frequency of the radiofrequency signal that is applied to the contact electrode unit, the deeper the generation depth of deep heat (that is, the higher the frequency of the radiofrequency signal, the shallower the generation depth of deep heat).

In FIG. 3, the first contact electrode 311 is shown as a positive electrode and the second contact electrode 312, which forms a pair with it, is shown as a negative electrode, but, when an alternating current by a radiofrequency signal is applied to the two contact electrodes, the polarities of both electrodes changes in response to the phase change of the alternating current. The same applies to the third contact electrode 321 and the fourth contact electrode 322.

As shown in FIG. 3, the high-frequency skin treatment device according to an embodiment of the present disclosure includes a multi-channel radio frequency generator 500 that includes a first channel 501 configured to transmit a radiofrequency signal to the contact electrode unit 300 generating deep heat in the deep region inside the skin, and a second channel 502 configured to transmit a radiofrequency signal to the plurality of needle electrodes for heating tissue inside the skin. The device may include a controller 400 that presets a plurality of treatment modes according to any one or a combination of non-invasive treatment using the contact electrode unit 300 and invasive treatment using the plurality of needle electrodes, and controls the needle driving motor 110 and the multi-channel radiofrequency generator 500 in accordance with a selected treatment mode among the plurality of treatment modes.

As shown in FIG. 3, the first channel 501 of the above-described multi-channel radiofrequency generator 500 may include a first radiofrequency generator 510 that generates a plurality of radiofrequency signals of different frequencies, depending on the generation depth of deep heat in a deep region inside the skin, and a switching module 512 that switches between radiofrequency signals such that a radiofrequency signal of a frequency selected from the plurality of radiofrequency signals generated by the first radiofrequency generator 510 is transmitted to the contact electrode unit 300

As shown in FIG. 3, the first radiofrequency generator 510 can generate a first frequency, a second frequency, and a third frequency of different frequencies, and the switching module 512 can selectively switch the first to third frequencies in accordance with the generation depth of deep heat under and transmit the switched frequencies to the contact electrode unit the control of the controller 400.

For example, when the magnitude of the frequencies of the radiofrequency signals generated by the first radiofrequency generator 510 is first frequency < second frequency < third frequency, the controller 400 first controls the switching module 512 such that the first frequency is connected to the contact electrode unit, thereby generating deep heat in a first depth region (a depth corresponding to the first frequency) of the skin; then, the switching module 512 switches the frequency to connect the second frequency to the contact electrode part, thereby generating deep heat in a second depth region (a depth corresponding to the second frequency); and the switching module 512 switches the frequency to connect the third frequency to the contact electrode unit, thereby generating deep heat in a third depth region (a depth corresponding to the third frequency) (here, the generation depth of deep heat varies depending on the magnitude of the frequency, and the depth of the first depth region > the depth of the second depth region > the depth of the third depth region.)

As described above, the contact electrode unit 300 generates, in contact with the skin, deep heat in a deep region of the skin using a radiofrequency signal, and can adjust the generation depth of deep heat by switching the signal in accordance with the magnitude of the frequency of the radiofrequency signal.

Meanwhile, as described above, while the contact electrode unit adjusts the generation depth of deep heat in accordance with the magnitude of the frequency, the plurality of needle electrodes of the needle assembly provided in the needle tip can adjust the skin penetration depth of the plurality of needle electrodes by the needle driving unit controlling the movement distance of the needle assembly under the control of the control unit. After inserting the needle electrodes to a desired depth inside the skin, the second radiofrequency generator of the second channel 502 transmits a radiofrequency signal to the plurality of needle electrodes, so that the plurality of needle electrodes delivers thermal energy to the tissue at the insertion depth, whereby tissue heating can be performed at the selected depth.

The plurality of needle electrodes 224, as shown in FIG. 3, may be configured such that adjacent needle electrodes have different polarities from each other, and when alternating current by a radiofrequency signal is applied to each of the needle electrodes, the polarity of each needle electrode changes in response to the phase change of the alternating current, whereby an electric field is generated between adjacent needle electrodes having different polarities, and heat is applied to the tissue between the adjacent needle electrodes.

The high-frequency skin treatment device according to an embodiment of the present disclosure can provide a non-invasive treatment mode that performs non-invasive treatment for generating deep heat in a deep region of the skin using the contact electrode unit, the first radiofrequency generator, and the switching module 512 described above, an invasive treatment mode that performs invasive treatment for heating the tissue in the skin using the needle driving unit, the plurality of needle electrodes, and the second radiofrequency generator, and a composite treatment mode that performs non-invasive treatment and invasive treatment in combination. The combined treatment mode may include a single-depth combined mode and a multi-depth combined mode. More specific details of the single-depth combined mode and the multi-depth combined mode will be described later.

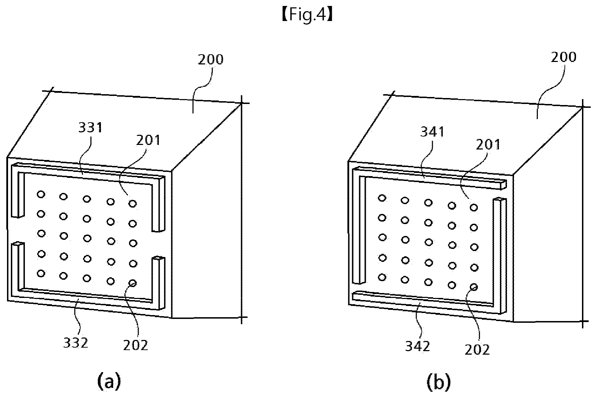

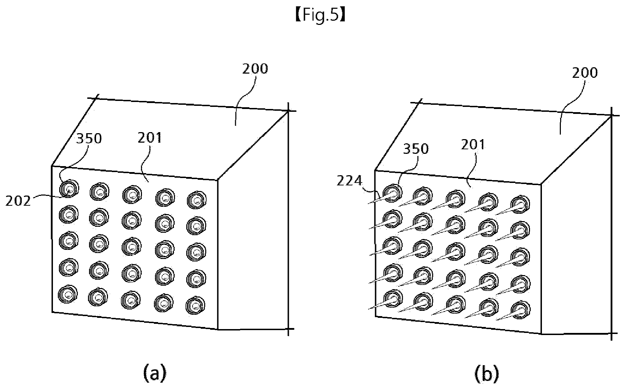

Meanwhile, various configurations of the contact electrode unit are described with reference to FIGS. 4 and 5.

In FIG. 4(a), the first contact electrode 331 and second contact electrode 332 protrude from the bottom surface 201 of needle tip 200 and are formed around the plurality of needle holes 202 in the shapes of "["and "]", respectively, and form an electrode pair. In FIG. 4(b), the first contact electrode 341 and the second contact electrode 342 are protruded and formed in the shapes of "¬" and reversed "∟", respectively, and form an electrode pair.

In the composite treatment mode, deep heat is generated inside the skin by the electrode pairs 331, 332 and 341, 342 of the contact electrode unit, thereby forming a deep heat region, and after preheating the tissue by the deep heat in this way, when the electrode pairs of the needle electrodes 224 penetrate the deep heat region and heat the tissue through radiofrequency energy, there is an advantage that the impedance of the skin tissue is lowered during invasive treatment and the delivery efficiency of the radiofrequency energy is increased, thereby enabling a comfortable procedure.

The contact electrode unit provided in the needle tip 200 shown in FIG. 5 includes a plurality of individual contact electrodes 350 that is individually provided for a plurality of needle holes 202, respectively, and provided for contact around the positions of the respective needle electrodes 224 that penetrate a treatment surface of the skin. FIG. 5(a) shows a state before the needle electrodes protrude through the needle holes 202 and FIG. 5(b) shows a state in which the needle electrodes 224 protrude through the needle holes 202.

As shown in FIG. 5, each individual contact electrode 350 is provided at a position corresponding to each needle electrode 224, and, like adjacent needle electrodes having different polarities, adjacent individual contact electrodes also have different polarities, so that the adjacent individual contact electrodes can form an electric field using a radiofrequency signal to generate deep heat.

As shown in FIG. 5, when an individual contact electrode 350 is provided at the position of each needle electrode, the region in which deep heat is generated in the skin by the individual contact electrodes 350 of each electrode pair can be limited to the region in which tissue heating is performed by the needle electrodes forming an electrode pair at that position, without widely spreading to the surrounding area. Accordingly, tissue can first be preheated by generation of deep heat in the skin tissue, and thermal coagulation is generated due to tissue heating by the needle electrodes in the deep-heat generation region, whereby the efficiency of radiofrequency energy delivery can be further improved.

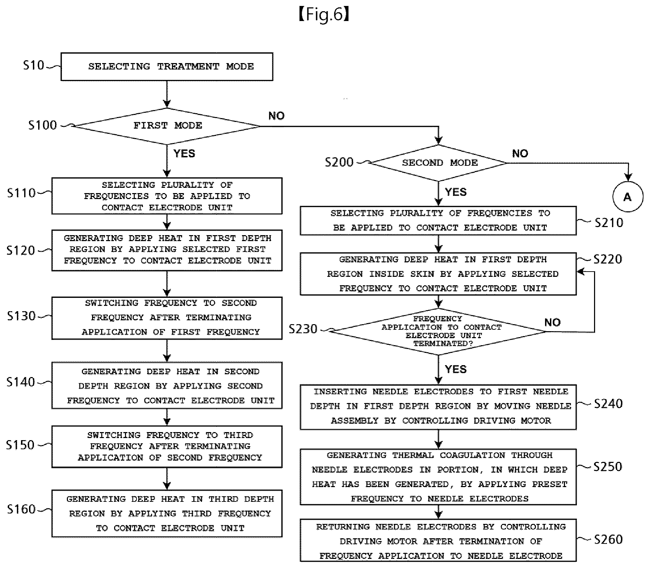

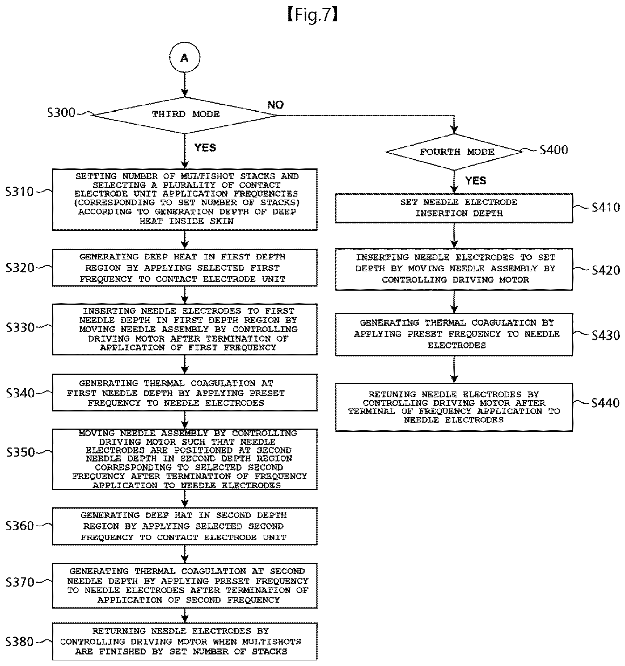

Meanwhile, a method of controlling the high-frequency skin treatment device according to an embodiment of the present disclosure is described with reference to the flowcharts shown in FIGS. 6 and 7. FIGS. 6 and 7 show the operation process for each of the treatment modes provided by the high-frequency skin treatment device according to an embodiment of the present disclosure, and FIGS. 6 and 7 are connected to each other on the basis of "A" in the figures, thereby representing an entire single flowchart.

The high-frequency skin treatment device according to an embodiment of the present disclosure provides four treatment modes of a first mode to a fourth mode, and any one of the four treatment modes can be selected in consideration of a practitioner's diagnosis or a patient's condition (S10).

The first mode S100 is a non-invasive treatment mode that performs non-invasive treatment that generates deep heat in a deep region of the skin using the contact electrode unit, the first radiofrequency generator, and the switching module, and the second mode and the third mode are composite treatment modes that perform both non-invasive treatment and invasive treatment in combination. The second mode S200 is a single-depth combined mode, the third mode S300 is a multi-depth combined mode, and the fourth mode S400 is an invasive treatment mode that performs invasive treatment that heats tissue in the skin using the needle driving unit, the plurality of needle electrodes, and the second radiofrequency generator.

When the first mode is selected (S100) a plurality of frequencies to be applied to the contact electrode unit may be selected by a practitioner in correspondence with the generation depth of deep heat (S110). In this case, the selected frequencies are referred to as a first frequency, a second frequency, and a third frequency.

In a state in which the contact electrode unit is in contact with the skin, the first frequency selected first is applied to the contact electrode unit, whereby deep heat is generated in a first depth region of the skin that corresponds to the first frequency (S120). That is, the controller controls the switching module such that the first frequency among a plurality of frequencies generated by the first radiofrequency generator is connected to the contact electrode unit, whereby deep heat can be generated in the first depth region.

After termination of application of the first frequency to the contact electrode unit, the controller controls the switching module to switch the frequency to the second frequency such that the second frequency is connected to the contact electrode unit (S130).

The contact electrode unit to which the second frequency is applied can generate deep heat in a second depth region of the skin that corresponds to the second frequency (S140).

After termination of application of the second frequency to the contact electrode unit, the controller controls the switching module to switch the frequency to the third frequency such that the third frequency is connected to the contact electrode unit (S150).

The contact electrode unit to which the third frequency is applied can generate deep heat in a third depth region of the skin that corresponds to the third frequency (S160).

In this way, by generating deep heat in various depth regions of the skin using the contact electrode unit, non-invasive treatment can be performed on a treatment surface of the skin.

On the other hand, when the second mode, which is a single-depth combined mode, is selected (S200), a frequency corresponding to the depth in the skin that requires treatment, that is, a frequency to be applied to the contact electrode unit is selected (S210), and the selected frequency is applied to the contact electrode unit with the contact electrode unit in contact with the treatment surface of the skin, so that deep heat can be generated in the first depth region of the skin (S220).

As described above, the generation of deep heat in the first depth region through the contact electrode unit is achieved by applying a frequency to the contact electrode unit for a preset time or for a time set by a practitioner.

After termination of application of a frequency to the contact electrode unit (S230), the controller operates and controls a driving motor of the needle driving unit to move the needle assembly, whereby the needle electrodes are inserted to a first needle depth in the first depth region (S240). Further, a thermal coagulation region can be formed in the region where deep heat has been generated, due to tissue heating through the needle electrodes by applying a preset frequency to the needle electrodes (S250).

That is, the needle electrodes are inserted into the preheated tissue of the first depth region, which is the region where the deep heat generated by the contact electrode unit has acted, thermal coagulation is generate by radiofrequency energy.

In this way, by generating deep heat in tissue of the skin through the contact electrode unit, the impedance of the skin tissue is lowered and the delivery efficiency of radiofrequency energy delivered by the needle electrodes is increased, which alleviates fear and pain associated with invasive treatment and enables a comfortable procedure.

The procedure according to the single-depth combined mode described above can control the frequency of a radiofrequency signal, which is applied to the contact electrode unit, and the insertion depth of the needle electrodes by the needle driving unit such that deep heat and thermal coagulation can be generated at a selected depth or a preset depth.

That is, when the insertion depth of the needle electrodes is set (or selected) as a first needle depth, the frequency that is applied to the contact electrode unit can be adjusted to the first frequency such that the contact electrode unit generates deep heat in a predetermined region including the first needle depth. When the insertion depth of the needle electrodes is set (or selected) as a second needle depth, the frequency that is applied to the contact electrode unit can be adjusted to the second frequency such that the contact electrode unit generates deep heat in a predetermined region including the second needle depth.

After generating thermal coagulation by inserting needle electrodes applying a radiofrequency, as described above, the controller can terminate the frequency application to the needle electrodes and control the driving motor such that the needle electrodes return to their original positions (S260).

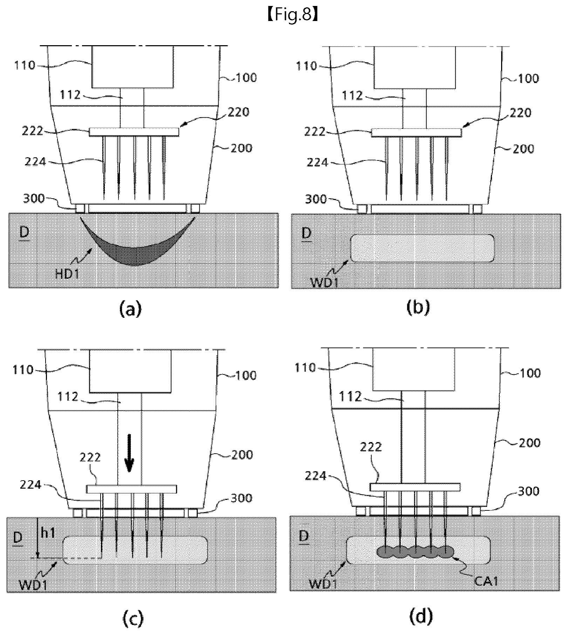

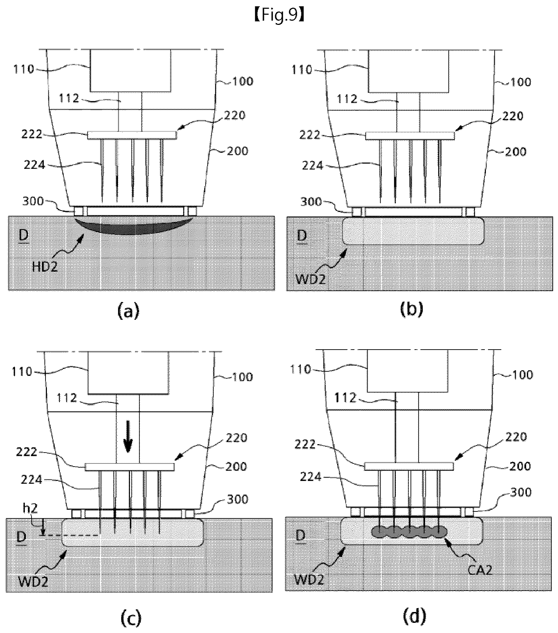

The procedure according to the single-depth combined mode described above is described in more detail with reference to FIGS. 8 and 9.

FIG. 8 shows a case in which deep heat is generated in a deep position in the skin and thermal coagulation is generated by needle electrodes, and FIG. 9 shows a case in which deep heat is generated in a shallower position of the skin than that shown in FIG. 8 and thermal coagulation is generated by the needle electrodes.

That is, the depth at which the needle electrodes penetrate the skin may be selected and determined in advance by a practitioner or may be set in advance, and assuming that a first needle depth is located deeper than a second needle depth, FIG. 8 shows, as a process for inserting the needle electrodes to the first needle depth to deliver radio frequency energy to the tissue inside the skin and to generate thermal coagulation, the preheating of a predetermined region including the first needle depth (referred to as a "first depth region") by delivering deep heat to the predetermined region including the first needle depth through the contact electrode unit before inserting the needle electrodes. Further, FIG. 9 shows , as a process of inserting the needle electrodes to the second needle depth (a shallower depth than the first needle depth) to deliver radiofrequency energy to tissue inside the skin and generate thermal coagulation, the preheating of a predetermined region including the second needle depth (referred to as a "second depth region") by delivering deep heat to the predetermined region including the second needle depth through the contact electrode unit before inserting the needle electrodes.

As shown in FIG. 8(a), in a state in which the contact electrode unit 300 provided at the end of the needle tip 200 is in contact with the skin surface, the controller controls the switching module such that the first frequency among a plurality of frequencies generated by the first radiofrequency generator is connected to the contact electrode unit 300, whereby deep heat HD1 can be delivered to tissue D inside the skin.

Since the deep heat HD1 is delivered to tissue D for a preset time or a time selected by a practitioner, as shown in FIG. 8(b), the first depth region WD1 becomes preheated and the generation of deep heat by the contact electrode unit 300 is terminated.

Subsequently, as shown in FIG. 8(c), the controller operates the driving motor 110, whereby the driving shaft 112 moves the needle assembly downward and the needle electrodes 224 pass through the needle holes and are inserted into the skin. In this process, under the control of the driving motor by the controller, the needle electrodes 224 are inserted to the first needle depth h1 in the first depth region WD1 preheated by the deep heat.

The controller, as shown in FIG. 8(d), controls the second radiofrequency generator to transmit a radiofrequency signal to the needle electrodes 224, and accordingly, tissue heating by the radiofrequency energy is performed near the ends of the needle electrodes 224, whereby thermal coagulation CA1 is generated.

Such thermal coagulation CA1 is generated near the first needle depth h1 in the first depth region WD1 preheated by the deep heat of the contact electrode unit.

As described above, by preheating the tissue D by generating deep heat by means of the contact electrode unit in the first depth region WD1 in the skin, the impedance of the skin tissue is lowered, and by inserting the needle electrodes to the first needle depth h1 in the first depth region WD1 and delivering radiofrequency energy, the energy delivery efficiency to the tissue can be increased, whereby it is possible to alleviate fear and pain due to invasiveness and enable a comfortable procedure.

Meanwhile, not only can the procedure be performed at a specific depth as described above, but it can also be performed continuously at other depths, and after generating thermal coagulation CA1 at the first needle depth h1, a procedure for generating thermal coagulation at the second needle depth, which is shallower than the first needle depth as shown in FIG. 9, can be performed.

As shown in FIG. 9(b), in a state in which the contact electrode unit 300 provided at the end of the needle tip 200 is in contact with the skin surface, the controller controls the switching module such that the second frequency among a plurality of frequencies generated by the first radiofrequency generator is connected to the contact electrode unit 300, whereby deep heat HD2 can be delivered to the tissue D inside the skin.

As described above, since the lower the frequency, the deeper the depth at which deep heat is generated, and conversely, the higher the frequency, the shallower the depth at which deep heat is generated, the contact electrode unit delivers deep heat to the first depth region inside the skin by the first frequency in FIG. 8, and, in FIG. 9, it is possible to deliver deep heat to the second depth region WD2, which is at a shallower depth than the first depth region, by applying the second frequency higher than the first frequency to the contact electrode unit.

Since the deep heat HD2 is delivered to tissue D for a preset time or a time selected by a practitioner, as shown in FIG. 9(b), the second depth region WD2 becomes preheated and the generation of deep heat by the contact electrode unit 300 is terminated.

Subsequently, as shown in FIG. 9(c), the controller operates the driving motor 110 whereby the driving shaft 112 moves the needle assembly downward and the needle electrodes 224 pass through the needle holes and are inserted into the skin. In this process, under the control of the driving motor by the controller, the needle electrodes 224 are inserted to the second needle depth h2 in the second depth region WD2 preheated by the deep heat.

The controller, as shown in FIG. 9(d), controls the second radiofrequency generator to transmit a radiofrequency signal to the needle electrodes 224, and accordingly, tissue heating by the radiofrequency energy is performed near the ends of the needle electrodes 224, whereby thermal coagulation CA2 is generated.

Such thermal coagulation CA2 is generated near the second needle depth h2 in the second depth region WD2 preheated by the deep heat of the contact electrode unit.

As described above, by preheating the tissue D by generating deep heat by means of the contact electrode unit in the second depth region WD2 in the skin, the impedance of the skin tissue is lowered, and by inserting the needle electrodes to the second needle depth h2 in the second depth region WD2 and delivering radiofrequency energy, the energy delivery efficiency to the tissue can be increased, whereby it is possible to alleviate fear and pain due to invasiveness and enable a comfortable procedure.

Meanwhile, the third mode is a multi-depth combined mode and the fourth mode that is for invasive treatment are described with reference to the flowchart shown in FIG. 7.

The multi-depth combined mode is a multi-shot procedure that generates deep heat and generates thermal coagulation through needle electrodes at each of a plurality of depths in the skin (when one shot is defined as thermal coagulation generation at one depth, multi-shot means generating thermal coagulation at a plurality of depths), and the number of depths at which shots are performed is defined as a "stack number". For example, when the stack number is 2, it means that shots are performed at two depths in the skin.

When the third mode, which is a multi-depth combined mode, is selected (S300), the multi-shot stack number is set, and a plurality of application frequencies of the contact electrode unit corresponding to the generation depth of deep heat in the skin may be selected in correspondence with the set stack number (S310).

For example, when the stack number is set to 2, a procedure is performed at two layers of needle depths in the skin, and for this purpose, two corresponding frequencies may be selected such that the contact electrode unit can generate deep heat in depth regions respectively corresponding to the two layers of needle depths.

Herein, a case in which the contact electrode unit generates deep heat in a first depth region including a first needle depth and a second depth region including a second needle depth such that the needle electrodes perform shots at the first needle depth and the second needle depth, and the first frequency and the second frequency are selected from a plurality of frequencies generated by the first radiofrequency generator for such generation of deep heat is described as an example.

In a state in which the contact electrode unit of the needle tip is in contact with the skin, in a first channel of the multi-channel radiofrequency generator, the controller can perform control such that the first frequency among the selected frequencies is connected to the contact electrode unit by the switching module, and the contact electrode unit to which the first frequency is applied can generate deep heat in the first depth region in the skin corresponding to the first frequency (S320).

The generation of deep heat in the first depth region through the contact electrode unit is achieved by applying a frequency to the contact electrode unit for a preset time or for a time set by a practitioner, and after the frequency application to the contact electrode unit is terminated, the controller operates and controls a driving motor of the needle driving unit to move the needle assembly, whereby the needle electrodes are inserted to the first needle depth in the first depth region (S330). Then, a preset frequency is applied to the needle electrodes, whereby a coagulation region can be formed in the region where deep heat has been generated, due to tissue heating through the needle electrodes (S340).

That is, the needle electrodes are inserted into the preheated tissue of the first depth region, which is the region where the deep heat generated by the contact electrode unit has acted, and thermal coagulation is formed by radiofrequency energy.

When the frequency application to the needle electrodes is terminated, one of the two stacks is completed, and for the procedure for the next layer, the controller can control the driving motor to move the needle assembly such that the needle electrodes are positioned at the second needle depth (S350).

That is, it is possible to move the needle electrodes to the second need depth without returning immediately after the needle electrode performs a shot at the first needle depth.

After the needle electrodes are moved to the second needle depth, in the first channel of the multi-channel radiofrequency generator, the controller can control the switching module to switch the frequency to the second frequency such that the second frequency is connected to the contact electrode unit, and the contact electrode unit to which the second frequency is applied can generate deep heat in the second depth region in the skin corresponding to the second frequency (S360).

The generation of deep heat in the second depth region through the contact electrode unit is achieved by applying a frequency to the contact electrode unit for a preset time or for a time set by a practitioner, and after the frequency application to the contact electrode unit is terminated, the controller applies a preset frequency to the needle electrodes, whereby a thermal coagulation region can be formed at the portion where the deep heat has been generated, due to tissue heating through the needle electrode (S370).

That is, in the preheated tissue of the second depth region, which is the region where the deep heat generated by the contact electrode unit has acted, the needle electrodes already positioned at the second needle depth heat the tissue using radiofrequency energy, whereby thermal coagulation can be generated.

After generating thermal coagulation by inserting needle electrodes and applying a radiofrequency at a plurality of a set stack number, as described above, the controller can terminate the frequency application to the needle electrodes and control the driving motor such that the needle electrodes return to their original positions (S380).

As described above, by allowing deep heat generation by the contact electrode unit and thermal coagulation generation by the needle electrodes to be continuously performed at multiple depths in the skin, it is possible not only to improve the efficiency of energy delivery and to alleviate fear and pain caused by invasiveness, thereby enabling a comfortable procedure, but also to considerably shorten the procedure time compared to when a procedure is performed separately for each depth.

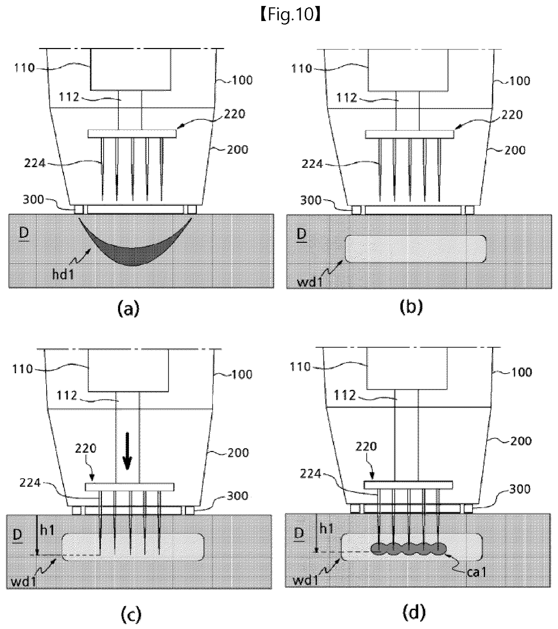

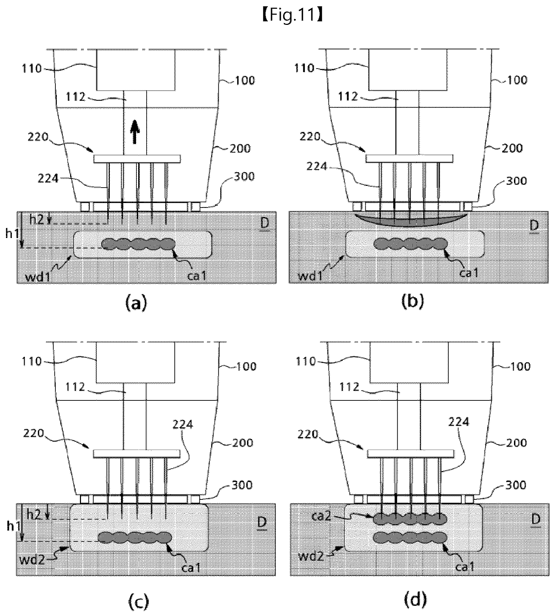

The procedure according to the multi-depth combined mode described above is described in more detail with reference to FIGS. 10 and 11.

FIGS. 10 and 11 illustrate an example of the procedure of the multi-depth combined mode according to the above-described steps S310 to S380, and although the procedural steps according to the multi-depth combined mode are separately shown in FIGS. 10 and 11, FIGS. 10 and 11 as a whole represent the process of a procedure.

That is, the multi-depth combined mode according to the steps S310 to S380 described above represents the process of a procedure that is performed in the order of FIG. 10(a) → FIG. 10(b) → FIG. 10(c) → FIG. 10(d) → FIG. 11(a) → FIG. 11(b) → FIG. 11(c) → FIG. 11(d).

The number of multishot stacks may be set to two, and the frequency that is applied to the contact electrode unit according to the generation depth of deep heat in the skin may be selected as a first frequency and a second frequency corresponding to the set stack number. First, as shown in FIG. 10(a), in a state in which the contact electrode unit 300 provided at the end of the needle tip 200 is in contact with the skin surface, the controller controls the contact electrode unit to connect the first frequency to the contact electrode unit 300, whereby a first deep heat hd1 can be delivered to the tissue D in the skin.

Since the first deep heat hd1 is generated by the contact electrode unit 300, as shown in FIG. 10(b), a first depth region wd1 of the tissue D in the skin is preheated, and the generation of the first deep heat by the contact electrode unit 300 is terminated.

Subsequently, as shown in FIG. 10(c), the controller operates the driving motor 110, whereby the driving shaft 112 moves the needle assembly downward and the needle electrodes 224 pass through the needle holes and are inserted into the skin. In this process, under the control of the driving motor by the controller, the needle electrodes 224 are inserted to the first needle depth h1 in the first depth region wd1 preheated by the deep heat.

The controller, as shown in FIG. 10(d), controls the second radiofrequency generator to transmit a radiofrequency signal to the needle electrodes 224, and accordingly, tissue heating by the radiofrequency energy is performed near the ends of the needle electrodes 224, whereby thermal coagulation CA2 is generated at the first needle depth h1 in the first depth region wd1 preheated by the deep heat.

When the thermal coagulation ca1 is generated at the first needle depth h1, as described above, the delivery of radiofrequency energy through the needle electrode 224 is terminated, and subsequently, as shown in FIG. 11(a), the controller controls the driving motor 110 to move the needle assembly 220 such that the needle electrodes 224 are positioned at a second needle depth h2.

In that state, as shown in FIG. 11(b), the controller can control the switching module to switch the frequency to a second frequency such that the second frequency is connected to the contact electrode unit 300, and the contact electrode unit 300 to which the second frequency is applied can generate second deep heat hd2 in a second depth region in the skin corresponding to the second frequency.

In this way, since the second deep heat hd2 is generated and the second depth region of the tissue in the skin is preheated, as shown in FIG. 11(c), both the first depth region and the second depth region wd2 can be preheated.

After the delivery of deep heat to the second depth region, the application of the second frequency to the contact electrode unit 300 is terminated, and the controller, as shown in FIG. 11(d), controls the second radiofrequency generator to transmit a radiofrequency signal to the needle electrodes 224, and accordingly, tissue heating by the radiofrequency energy is performed near the ends of the needle electrodes 224, whereby thermal coagulation ca2 is generated at the second needle depth h2 in the second depth region wd2 preheated by the deep heat.

As described above, in the multi-depth combined mode, thermal coagulations ca1 and ca2 can be generated respectively at the first needle depth h1 and the second needle depth h2 through a single process, as shown in FIG. 11(d), thereby being able to complete a multishot.

FIGS. 10 and 11 shows a process of generating thermal coagulations respectively at two depths in accordance with two stacks, but the present disclosure is not limited thereto, and it is of course possible to generate deep heat and thermal coagulation at multiple depths in accordance with three or more stacks.

For example, in the multi-depth combined mode, three can be selected as the number of multishot stacks, and a first frequency, a second frequency, and a third frequency can be respectively selected as frequencies that are applied to the contact electrode unit.

Accordingly, a radiofrequency signal of the first frequency is applied to the contact electrode unit, whereby deep heat is generated in a first depth region in the skin, and the controller controls the needle driving unit to insert a plurality of needle electrodes to a first needle depth in a first depth region, and a radiofrequency signal is applied to the inserted plurality of needle electrodes to deliver heat to the tissue at the first needle depth, whereby thermal coagulation can be generated. Subsequently, the controller controls the needle driving unit to position the plurality of needle electrodes at a second needle depth in a second depth region corresponding to the second frequency, the frequency of the radiofrequency signal applied to the contact electrode unit is switched to the second frequency to generate heat in the second depth region in the skin, and a radiofrequency signal is applied to the plurality of needle electrodes positioned at the second needle depth to deliver heat to the tissue at the second needle depth, whereby thermal coagulation can be generated. Subsequently, the controller controls the needle driving unit to position the plurality of needle electrodes at a third needle depth in a third depth region corresponding to the third frequency, the frequency of the radiofrequency signal applied to the contact electrode unit is switched to the third frequency to generate heat in the third depth region in the skin, and a radiofrequency signal is applied to the plurality of needle electrodes positioned at the third needle depth to deliver heat to the tissue at the third needle depth, whereby thermal coagulation is generated. Accordingly, thermal coagulation is generated at three depths in the skin, whereby a multishot can be completed.

In this way, by allowing deep heat generation by the contact electrode unit and thermal coagulation generation by the needle electrodes to be continuously performed at multiple depths in the skin, the present disclosure has a distinct advantage that it is possible not only to improve the efficiency of energy delivery and to alleviate fear and pain caused by invasiveness, thereby enabling a comfortable procedure, but also to considerably shorten the procedure time compared to when a procedure is performed separately for each depth.

Meanwhile, returning to FIG. 7, when the fourth mode, which is an invasive treatment mode, is selected (S400), the insertion depth of the needle electrodes can be set by a practitioner (S410), and the controller controls the driving motor to move the needle assembly, whereby the needle electrodes can be inserted to the set depth (S420).

Further, in a state in which the needle electrodes are inserted to the set depth, the controller causes a preset frequency to be applied to the needle electrodes, whereby it is possible to heat the skin tissue at the insertion position and generate thermal coagulation (S430).

After the frequency application to the needle electrodes is terminated, the controller can control the driving motor such that the needle electrodes return to their original positions (S440).

As described above, the high-frequency skin treatment device according to an embodiment of the present disclosure, which is a high-frequency skin treatment device that performs radiofrequency skin treatment by delivering radiofrequency energy to the skin, and a method of controlling the high-frequency skin treatment device have distinctive advantages that it is possible to selectively or concurrently perform radiofrequency invasive treatment and non-invasive treatment in a single device; to improve the efficiency of radiofrequency energy delivery and alleviate the patient's fear and pain due to invasiveness to enable a more comfortable treatment; and to significantly reduce the procedure time by enabling multiple procedures at multiple depths in the skin in a single process.

### [Industrial Applicability]

The high-frequency skin treatment device according to the present disclosure, and the method of controlling the high-frequency skin treatment device can be utilized in the industrial field of skin aesthetics and skin treatment that make it possible to obtain effects such as reducing skin wrinkles and enhancing skin elasticity using radiofrequency.

## Claims

1. A high-frequency skin treatment device comprising:
a contact electrode unit configured to be brought into contact with a treatment surface of a skin and generate deep heat in a deep region of the treatment surface by applying a radiofrequency signal;
a needle assembly including a plurality of needle electrodes configured to penetrate the treatment surface and deliver heat to tissue using a radiofrequency signal;
a needle driving unit configured to control insertion of the plurality of needle electrodes by moving the needle assembly;
a multi-channel radiofrequency generator including a first channel configured to transmit a radiofrequency signal for generating the deep heat to the contact electrode unit, and a second channel configured to transmit a radiofrequency signal for heating the tissue to the plurality of needle electrodes; and
a controller configured to preset a plurality of treatment modes according to any one of or a combination of non-invasive treatment using the contact electrode unit and invasive treatment using the plurality of needle electrodes, and to control the needle driving unit and the multi-channel radiofrequency generator in accordance with selected treatment modes.

2. The high-frequency skin treatment device of claim 1, wherein the controller, in accordance with the treatment modes, is configured to control a generation depth of deep heat in the skin by the contact electrode unit by switching a radiofrequency signal by controlling the first channel of the multi-channel radiofrequency generator in the non-invasive treatment, and to control a tissue heating depth of the inserted needle electrodes by controlling an insertion depth of the plurality of needle electrodes into the skin by controlling the needle driving unit in the invasive treatment.

3. The high-frequency skin treatment device of claim 1, wherein the controller, in accordance with the treatment modes, controls the first channel of the multi-channel radiofrequency generator to transmit a radiofrequency signal of a frequency for generating deep heat by the contact electrode unit at a depth corresponding to an insertion depth of the needle electrodes selected or preset for the invasive treatment in the non-invasive treatment, and, after deep heat is generated by the contact electrode unit, controls the needle driving unit to insert the plurality of needle electrodes to the selected or preset insertion depth of the needle electrodes such that the invasive treatment is performed.

4. The high-frequency skin treatment device of claim 1, wherein the first channel of the multi-channel radiofrequency generator includes: a first radiofrequency generator configured to generate a plurality of radiofrequency signals of different frequencies, depending on a generation depth of deep heat in a deep region inside the skin; and a switching module configured to switch between radiofrequency signals such that a radiofrequency signal of a frequency selected from the plurality of radiofrequency signals generated by the first radiofrequency generator is transmitted to the contact electrode unit.

5. The high-frequency skin treatment device of claim 1, wherein the needle driving unit is configured to control a movement distance of the needle assembly under control of the controller such that an insertion depth of the plurality of needle electrodes into the skin can be adjusted;
the first channel of the multi-channel radiofrequency generator is configured to generate radiofrequency signals of a plurality of different frequencies depending on a generation depth of deep heat in the skin and to switch between the plurality of radiofrequency signals such that a radiofrequency signal of a selected frequency is transmitted to the contact electrode unit;
the second channel of the multi-channel radiofrequency generator is configured to generate a radiofrequency signal of a selected or preset frequency to transmit the radiofrequency signal to the plurality of needle electrodes such that the inserted plurality of needle electrodes delivers heat to tissue; and
the controller is configured to, when a frequency of the radiofrequency signal transmitted through the first channel is selected, perform control such that deep heat is generated in a depth region inside the skin corresponding to the selected frequency by the contact electrode unit, and to control the needle driving unit such that the plurality of needle electrodes is inserted to a needle insertion depth in the depth region, in which the deep heat has been generated, and the inserted needle electrodes transmit heat to the region in which the deep heat has been generated in accordance with the radiofrequency signal.

6. The high-frequency skin treatment device of claim 1, wherein the controller is configured to, when a plurality of different frequencies for the non-invasive treatment is selected, separately control the first channel of the multi-channel radiofrequency generator, the needle driving unit, and the second channel of the multi-channel radiofrequency generator to sequentially perform a combined treatment of generating deep heat through the contact electrode unit, inserting the plurality of needle electrodes, and heating tissue through the inserted needle electrodes, at each of different depths in the skin corresponding in number to the number of the selected frequencies.

7. The high-frequency skin treatment device of claim 1, wherein the needle driving unit is provided in a handpiece, and the needle assembly is provided in a needle tip detachably connected to an end of the handpiece to be connected to the needle driving unit;
the plurality of needle holes is formed on a bottom surface of the needle tip such that the plurality of needle electrodes protrudes through the needle holes, respectively; and
the contact electrode unit includes a first contact electrode and a second contact electrode that form an electrode pair around the plurality of needle holes on the bottom surface of the needle tip and protrude from the bottom surface of the needle tip.

8. The high-frequency skin treatment device of claim 1, wherein the needle driving unit is provided in a handpiece, and the needle assembly is provided in a needle tip detachably connected to an end of the handpiece to be connected to the needle driving unit;
the plurality of needle holes is formed in a bottom surface of the needle tip such that the plurality of needle electrodes protrudes through the needle holes, respectively; and
the contact electrode unit includes a plurality of individual contact electrodes provided for the plurality of needle holes, respectively, to respectively come into contact with the peripheries of positions of the needle electrodes inserted in the treatment surface.

9. A method of controlling a high-frequency skin treatment device, which provides treatment modes based on a combination of a non-invasive treatment for a skin that uses a contact electrode unit configured to come into contact with a treatment surface of the skin, and an invasive treatment that inserts a plurality of needle electrodes into the treatment surface and delivers heat to tissue in accordance with a radiofrequency signal, the method comprising:
selecting a frequency of a radiofrequency signal for the non-invasive treatment;
generating deep heat in a depth region inside the skin corresponding to the selected frequency by applying the radiofrequency signal of the selected frequency to the contact electrode unit that is in contact with the treatment surface;
controlling a needle driving unit to move the plurality of needle electrodes such that the plurality of needle electrodes are inserted to a needle depth in the depth region in which the deep heat has been generated; and
applying a preset or selected radiofrequency signal to the plurality of needle electrodes such that heat is delivered through the plurality of needle electrodes to tissue at the needle depth in the depth region in which the deep heat has been generated.

10. The method of claim 9, further comprising:
switching the frequency applied to the contact electrode unit to a radiofrequency signal of another frequency to change the depth region in which the deep heat has been generated; and
controlling the needle driving unit such that the plurality of needle electrodes is inserted to a needle depth in the depth region corresponding to the frequency changed by the switching.

11. A method of controlling a high-frequency skin treatment device, which provides treatment modes based on a combination of a non-invasive treatment for a skin that uses a contact electrode unit configured to come into contact with a treatment surface of the skin, and an invasive treatment that inserts a plurality of needle electrodes into the treatment surface and delivers heat to tissue in accordance with a radiofrequency signal, the method comprising:
setting the number of multi-shot stacks and selecting contact electrode unit application frequencies corresponding in number to the set number of the stacks; and
delivering a radiofrequency signal to the contact electrode unit while switching between a plurality of frequencies selected to generate deep heat in each of depth regions inside a skin corresponding to the set number of the stacks, setting a needle depth at which the plurality of needle electrodes is to be positioned, in each of the depth regions, and delivering heat according to a radiofrequency signal with the plurality of needle electrodes positioned at each of the needle depths under control of a needle driving unit.

12. The method of claim 11, wherein the delivering of heat according to a radiofrequency signal with the plurality of needle electrodes positioned at each of the needle depths sequentially performs a combined treatment of generating deep heat through the contact electrode unit, moving the plurality of needle electrodes to a corresponding needle position, and heating tissue through the inserted needle electrodes, in each of the depth regions.

13. The method of claim 11, wherein, in the selecting of contact electrode unit application frequencies, two are selected as the number of the stacks, and a first frequency and a second frequency are selected as the contact electrode unit application frequencies, and
the delivering of heat according to a radiofrequency signal with the plurality of needle electrodes positioned at each of the needle depths includes;
generating deep heat in a first depth region inside the skin by applying a radiofrequency signal of the first frequency to the contact electrode unit, inserting the plurality of needle electrodes to a first needle depth in the first depth region by controlling the needle driving unit, and delivering heat to tissue at the first needle depth by applying a radiofrequency signal to the inserted plurality of needle electrodes; and
positioning the plurality of needle electrodes at a second needle depth in a second depth region corresponding to the second frequency by controlling the needle driving unit, generating deep heat in the second depth region inside the skin by switching the frequency of a radiofrequency signal applied to the contact electrode unit to the second frequency, and delivering heat to tissue at the second needle depth by applying a radiofrequency signal to the plurality of needle electrodes positioned at the second needle depth.

14. The method of claim 11 wherein, in the selecting of contact electrode unit application frequencies, three are selected as the number of the stacks, and a first frequency, a second frequency, and a third frequency are selected as the contact electrode unit application frequencies, and
the delivering of heat according to a radiofrequency signal with the plurality of needle electrodes positioned at each of the needle depths includes:
generating deep heat in a first depth region inside the skin by applying a radiofrequency signal of the first frequency to the contact electrode unit, inserting the plurality of needle electrodes to a first needle depth in the first depth region by controlling the needle driving unit, and delivering heat to tissue at the first needle depth by applying a radiofrequency signal to the inserted plurality of needle electrodes;
positioning the plurality of needle electrodes at a second needle depth in a second depth region corresponding to the second frequency by controlling the needle driving unit, generating deep heat in the second depth region inside the skin by switching the frequency of a radiofrequency signal applied to the contact electrode unit to the second frequency, and delivering heat to tissue at the second needle depth by applying a radiofrequency signal to the plurality of needle electrodes positioned at the second needle depth; and
positioning the plurality of needle electrodes at a third needle depth in a third depth region corresponding to the third frequency by controlling the needle driving unit, generating deep heat in the third depth region inside the skin by switching the frequency of a radiofrequency signal applied to the contact electrode unit to the third frequency, and delivering heat to tissue at the third needle depth by applying a radiofrequency signal to the plurality of needle electrodes positioned at the third needle depth.
